# EUROPÄISCHE PATENTSCHRIFT

(11) **EP 3 714 840 B1**
(45) Veröffentlichungstag und Bekanntmachung des Hinweises auf die Patenterteilung: **24.08.2022**
(21) Anmeldenummer: 19211440.3
(22) Anmeldetag: 26.11.2019
(51) Int. Cl.: A61F 2/34

(54) **HÜFTPFANNE EINER HÜFTGELENK-ENDOPROTHESE**
ACETABULAR CUP FOR A HIP JOINT ENDOPROSTHESIS
CAVITÉ COTYLOÏDE D'UNE ENDOPROTHÈSE DE L'ARTICULATION DE LA HANCHE

(30) Priorität: 27.02.2019 EP 19159566
(43) Veröffentlichungstag der Anmeldung: 30.09.2020
(73) Patentinhaber: ARTIQO GmbH, 59348 Lüdinghausen (DE)
(72) Erfinder: Bücken, Ulrich, 59348 Lüdinghausen (DE); Frank, Thomas Mario, 7000 Eisenstadt (AT)
(74) Vertreter: Gosdin, Carstensen & Partner Patentanwälte Partnerschaftgesellschaft mbB

(56) Entgegenhaltungen:
- EP-A1- 0 444 382
- EP-A1- 2 193 764
- WO-A1-2008/015289
- WO-A2-2008/015285
- WO-A2-2008/015286
- DE-C1- 4 220 462
- US-A1- 2017 290 666

## Beschreibung

Die Erfindung betrifft eine Hüftpfanne einer Hüftgelenk-Endoprothese, umfassend einen äußeren Pfannenabschnitt mit einer konvexen Oberfläche zur Kontaktnahme mit dem Acetabulum und mit einem inneren Pfannenabschnitt mit einer konkaven Oberfläche zur Kontaktnahme mit einem Inlay der Hüftgelenk-Endoprothese, wobei zumindest über einen Oberflächenabschnitt der Hüftpfanne der äußere Pfannenabschnitt relativ zum inneren Pfannenabschnitt in eine radiale Richtung federelastisch gestaltet ist, wobei die Federelastizität durch einen Wandungsabschnitt des äußeren Pfannenabschnitts gebildet wird, der zu einem Wandungsabschnitt des inneren Pfannenabschnitts unter Bildung eines Hohlraums mit radialem Abstand verläuft, wobei im Hohlraum mindestens ein federelastisches Element angeordnet ist, welches eine Federwirkung in radiale Richtung zwischen dem Wandungsabschnitt des äußeren Pfannenabschnitts und dem Wandungsabschnitt des inneren Pfannenabschnitts erzeugt.

Eine gattungsgemäße Hüftpfanne wird in der EP 0 444 382 A1 und in der DE 42 20 462 C1 offenbart. Die in letzterem Dokument offenbarte Hüftpfanne weist eine Außenschale, einen Einsatz aus Kunststoffmaterial in der Außenschale und Stoßdämpfungsmittel zwischen der Außenschale und dem Einsatz auf. Der Einsatz ist von einer Innenschale ummantelt, wobei die Stoßdämpfungsmittel zwischen der Innenschale und der Außenschale angeordnet sind. Ähnliche und andere Lösungen zeigen die US 2017/0290666 A1, die WO 2008/015285 A2, die WO 2008/015289 A1, die EP 2 193 764 A1 und die WO 2008/015286 A2. Zur generellen Technologie sei weiter auf die EP 0 222 159 A2 hingewiesen.

Im Englischen wird die Hüftpfanne als "cup" oder "shell" bezeichnet und besteht zumeist aus Metall. Sie wird in das Acetabulum, d. h. in die natürliche Hüftpfanne des Beckenknochens, implantiert, wobei der Aufnahmebereich für die Hüftpfanne kugelabschnittsartig vorgefräst werden kann. Der äußere Pfannenabschnitt mit seiner konvexen Oberfläche ist demgemäß im implantierten Zustand im Kontakt mit dem Beckenknochen, während der innere Pfannenabschnitt mit seiner (im Wesentlichen) konkaven Fläche einen Aufnahmeraum für ein Inlay der Prothese bildet.

Das Inlay wird in die Konkavität der zumeist metallischen Hüftpfanne eingesetzt und mit dieser über eine Inlaykupplung oder Innenkupplung kraftschlüssig verbunden. Es nimmt den metallischen oder keramischen Hüftkopf des femoralen Teils der Hüftprothese auf.

Dabei ist vorgesehen, dass die Prothesen-Hüftpfanne mit Übermaß ("Pressfit") in das Acetabulum implantiert, d. h. in dieses eingeschlagen wird. Daher besitzen üblicherweise zu implantierende Hüftpfannen im Durchmesser ein Übermaß von ca. 2 % bis 3 % gegenüber dem gefrästen Acetabulum. Dies bedeutet, dass beispielweise eine Hüftpfanne mit einer Größe von 50 mm einen effektiven Durchmesser von beispielsweise 51 mm besitzt.

Demgemäß wird durch das Übermaß beim Einschlagen der Hüftpfanne eine gewünschte Vorspannung (d. h. der "Pressfit") erzeugt. Die zumeist metallische Hüftpfanne soll sich beim Einschlagen im knöchernen, ausgefrästen Acetabulum möglichst gleichmäßig verspannen, ohne dass an der Innenkontur ein nennenswerter Verzug, beispielsweise eine ovale Entrundung, eintritt.

Der Pressfit ist gemeinsam mit einer möglichst rauen (konvexen äußeren) Oberfläche der Hüftpfanne für die Primärstabilität der künstliche Hüftpfanne verantwortlich. Das Pressfit ist im Bereich des Pfannenrandes am stärksten und nimmt zum Pfannenboden (d. h. zum Pol) hin ab.

Die Sekundärstabilität der Hüftpfanne wird nach 6 bis 12 Wochen durch das Einwachsen von Knochen in die raue Oberfläche erreicht, gleichzeitig wird die Vorspannung (d. h. der Pressfit) durch Umbauvorgänge des Knochens abgebaut.

Bekannt ist es, dass Pressfitpfannen modular aufgebaut sind. Dies bedeutet, dass in die metallische Hüftpfanne (mittels einer konischen oder sphärischen Kupplung) Inlays aus Kunststoff und/oder Keramik eingebracht werden können. Die konischen Innenkupplungen unterliegen (vor allem für die Aufnahme von keramischen Inlays) hohen Genauigkeiten in Bezug auf Winkeltoleranzen, Durchmesser und Rundheit.

Da die Inlays nach dem Einschlagen in die Pfanne eingesetzt werden (es besteht eine entsprechende Auswahlmöglichkeit verschiedener Inlaytypen je nach intraoperativer Situation), darf der Einschlagvorgang einschließlich des Pressfit die geforderte Genauigkeit der Inlaykupplung nicht beeinträchtigen. Gleichzeit ist beim Einschlagen der Hüftpfanne ein gutes Setzverhalten derselben gefordert. Dies wird (neben der Außengeometrie) von der Wandstärke der Hüftpfannen-Schale beeinflusst.

Dünnwandig Pfannen mit einem elastischen Verhalten der Schale (Federwirkung) erreichen das angestrebte günstigere Setzverhalten gegenüber dickwandigen bzw. steifen Pfannen. Unterschiede in der Knochenhärte können auch besser toleriert werden.

Ein Sortiment von Pressfitpfannen umfasst meist zehn Größen von Durchmesser 46 bis 64. Um das Inlaysortiment so klein wie möglich zu halten, werden oft zwei oder mehrere Hüftpfannen-Kopfgrößen mit einer äußeren Inlaykontur ausgestattet. Dies führt zu variierenden Wandstärken innerhalb eines Pfannensystems, so dass sich ein größenspezifisches Einschlagverhalten ergibt. Des Weiteren haben verschiedene Pfannensteifigkeiten in Zusammenhang mit variierenden Durchmessern einen Einfluss auf die Spannung im umgebenden Knochen und eventuell auf die Umbauvorgänge des Knochengewebes.

Der vorliegenden Erfindung liegt die **Aufgabe** zugrunde, eine Hüftpfanne der eingangs genannten Art so weiterzubilden, dass diese ein weiter verbessertes einheitliches Setz- bzw. Einschlagverhalten und eine Formstabilität des konkaven inneren Pfannenabschnitts über das gesamte zum Einsatz kommende Größenspektrum aufweist. Demgemäß soll sich eine beim Setzen und/oder Einschlagen erfolgende Formänderung des äußeren Pfannenabschnitts nicht unmittelbar auf die Kontur des inneren Pfannenabschnitts übertragen.

Die **Lösung** dieser Aufgabe durch die Erfindung ist dadurch gekennzeichnet, dass das federelastische Element durch eine Anzahl Federlamellen gebildet wird, die am einen Wandungsabschnitt angeformt sind und die auf der Oberfläche des anderen Wandungsabschnitts anliegen, wobei die Federlamellen um den Umfang des Wandungsabschnitts umlaufend angeordnet sind, wobei um den Umfang des Wandungsabschnitts zwischen 14 und 40 Federlamellen angeordnet sind, wobei die Federlamellen, aus radialer Richtung betrachtet, eine rechteckige oder trapezförmige Grundkontur aufweisen und wobei die Federlamellen aus dem Material eines Wandungsabschnitts direkt ausgeformt sind oder dass das federelastische Element in einem Radialschnitt eine waben- oder fachwerkartige Struktur aufweist.

Die Federlamellen sind dabei bevorzugt am Wandungsabschnitt des inneren Pfannenabschnitts angeformt und liegen auf der Oberfläche des Wandungsabschnitts des äußeren Pfannenabschnitts an.

Dabei ist insbesondere vorgesehen, dass um den Umfang des Wandungsabschnitts zwischen 16 und 35 Federlamellen angeordnet sind.

In vertikale Richtung der Hüftpfanne versetzt können auch mindestens zwei Reihen Federlamellen angeordnet sein.

Die Federlamellen sind dabei bevorzugt mit ihrem in vertikale Richtung der Hüftpfanne oberen oder unteren Endbereich mit dem Wandungsabschnitt verbunden.

Die Federlamellen treten bevorzugt unter einem Winkel zwischen 15° und 45°, vorzugsweise zwischen 20° und 35°, zur Oberfläche des Wandungsabschnitts aus diesem aus. Im Übergangsbereich zwischen Wandungsabschnitt und Federlamelle kann weiterhin eine Ausrundung ausgebildet sein; diese hat vorzugsweise einen Radius zwischen 0,1 mm und 1,5 mm, besonders bevorzugt zwischen 0,2 mm und 1,0 mm.

In Umfangsrichtung des Wandungsabschnitts kann zwischen mindestens zwei Federlamellen ein sich in vertikale Richtung erstreckendes Versteifungselement am Wandungsabschnitt angeordnet sein. Das Versteifungselement hat dabei auch den Vorteil, dass er eine Verdrehsicherung zwischen den Teilen herstellt, aus denen die Hüftpfanne gegebenenfalls besteht.

Da sich außerdem im eingeschlagenen Zustand der elastische Oberflächenabschnitt des äußeren Pfannenabschnitts nach innen biegt, stehen die durch die Versteifungselemente abgestützten Abschnitte etwas vor und bilden insoweit eine Verdrehsicherung für die Hüftpfanne auch im acetabulären Knochen.

Eine weitere Fortbildung der Erfindung sieht vor, dass sich der federelastisch gestaltete Oberflächenabschnitt auf eine Höhe beschränkt, die maximal 60 %, vorzugsweise maximal 50 %, der gesamten Höhe der Hüftpfanne beträgt.

Der federelastisch gestaltete Oberflächenabschnitt verläuft bevorzugt um den gesamten Umfang der Hüftpfanne.

Die Hüftpfanne besteht bevorzugt aus Metall.

Dabei ist insbesondere vorgesehen, dass sie durch einen additiven Fertigungsprozess hergestellt ist. Hierunter ist insbesondere, aber nicht ausschließlich, der 3-D-Druck zu verstehen, mit dem es in besonders vorteilhafter Weise möglich ist, die gesamte Struktur der Hüftpfanne einteilig bzw. aus nur wenigen Teilen zu fertigen und zwar insbesondere einschließlich der federelastischen Struktur bzw. Strukturen im genannten Hohlraum.

Somit ergibt sich vorteilhaft eine Außenschale der Hüftpfanne, die eine besondere nachgiebige Außenkontur aufweist, während die Innenschale der Hüftpfanne eine hohe Formstabilität und somit eine stabile Innenkontur aufweist.

Mit der vorgeschlagenen Lösung werden verschiedene Vorteile erreicht:
Die Hüftpfanne hat im Bereich der Pressfit-Wirkung eine vorteilhafte elastische Charakteristik (d. h. Federwirkung) an der Außenschale, so dass ein ideales Setzverhalten und eine einheitliche Spannung im Knochenlager über das gesamte Größenspektrum erreicht werden.

Die Innenkontur der Hüftpfanne ist für die Aufnahme von modularen Inlays geeignet. Somit sind die Anforderungen für Keramik- oder PE-Inlays erfüllt.

Die durch das Pressfit entstehenden Spannungen wirken nicht auf die Innenkontur; insbesondere werden keine Verformungen der Innenkontur hervorgerufen.

Weiterhin werden hohe Spannungsspitzen vermieden, welche in situ beispielsweise beim Laufen oder Stolpern auftreten können, so dass derartige Spannungsspitzen nicht vollständig auf die Inlay-Kupplung wirken.

Vorteilhaft wird, wie erläutert, ein additives Fertigungsverfahren (insbesondere der 3-D-Druck) eingesetzt, so dass eine einstückige Gestaltung (bzw. eine solche aus nur wenigen Teilen) der Hüftpfanne möglich ist. Hierdurch können die Vorteile einer elastischen Außenschale der Hüftpfanne mit den Anforderungen einer modularen Inlay-Kupplung kombiniert werden. Vorteilhaft ist bei der vorgeschlagenen Lösung weiterhin, dass unterschiedliche Größen von Hüftpfannen mit relativ gleichbleibender Steifigkeit gestaltet werden können.

Die Dimensionierung und Auslegung der federelastischen Elemente, d. h. der erfindungsgemäßen Federlamellen bzw. waben- oder fachwerkartigen Strukturen, kann in einfacher Weise fachmännisch durch den Einsatz von Finiten Elementen erfolgen und damit ohne bzw. vor der Implantation beim Patienten für jede modulare Größe separat optimiert werden.

Betreffend die Gestaltung, Dimensionierung und Auslegung der federelastischen Elemente und insbesondere der Federlamellen sei noch auf folgendes hingewiesen: Beim Einschlagen der Hüftpfanne in das Acetabulum entstehen entsprechende Kräfte, die die Hüftpfanne auf das Acetabulum ausübt. Dabei ist es generell wünschenswert, dass diese Kräfte gewisse Maximalwerte nicht übersteigen, um die Vorspannung in der Hüftpfanne im Acetabulum zu begrenzen. Daher kann vorgesehen werden, dass durch entsprechende Gestaltung und Dimensionierung der federelastischen Elemente diese, beispielsweise in ihren Ansatzbereich an der Hüftpfanne, geringfügig plastisch verformt werden, so dass die vom federelastischen Element auf das Acetabulum ausgeübten Kräfte limitiert sind.

In der Zeichnung sind Ausführungsbeispiele der Erfindung dargestellt. Es zeigen:
- Fig. 1: in einer Radialschnittdarstellung eine in das Acetabulum, d. h. in die Beckenpfanne, eingeschlagene Hüftpfanne, die Bestandteil einer Hüftgelenk-Endoprothese ist, wobei die Hüftpfanne hier zunächst noch schematisch dargestellt ist,
- Fig. 2: in einer Radialschnittdarstellung analog zu Figur 1 das Acetabulum mit an dieses angesetzter, aber noch nicht eingeschlagener Hüftpfanne, wobei die Hüftpfanne wiederum zunächst noch schematisch dargestellt ist,
- Fig. 3: in einer Radialschnittdarstellung die isolierte Hüftpfanne, die zunächst noch schematisch dargestellt ist,
- Fig. 4: in einer Radialschnittdarstellung die Hälfte einer ersten konkreten Ausführungsform der Hüftpfanne,
- Fig. 5: in der Vorderansicht einen inneren Teil der Hüftpfanne mit einer größeren Anzahl an Federlamellen,
- Fig. 6: in der Vorderansicht einen inneren Teil der Hüftpfanne mit einer geringeren Anzahl an Federlamellen,
- Fig. 7: eine vergrößerte Darstellung des unteren rechten Abschnitts des inneren Teils der Hüftpfanne gemäß Figur 4 und
- Fig. 8: in einer Radialschnittdarstellung einen Teil der Hüftpfanne nach einer anderen Ausführungsform der Erfindung, wobei eine waben- bzw. fachwerkartige Federstruktur zwischen zwei Wandungsabschnitten angeordnet ist.

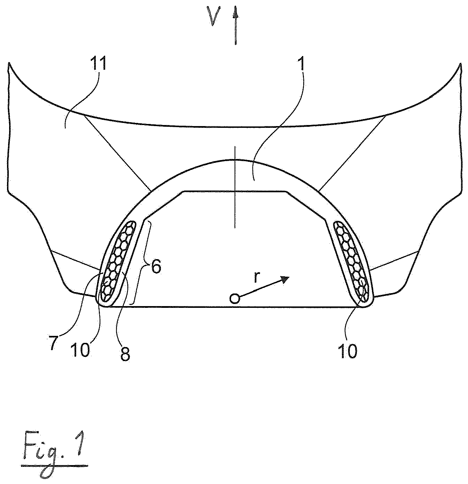

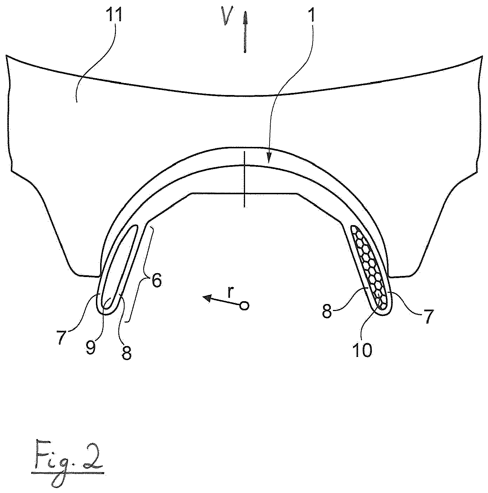

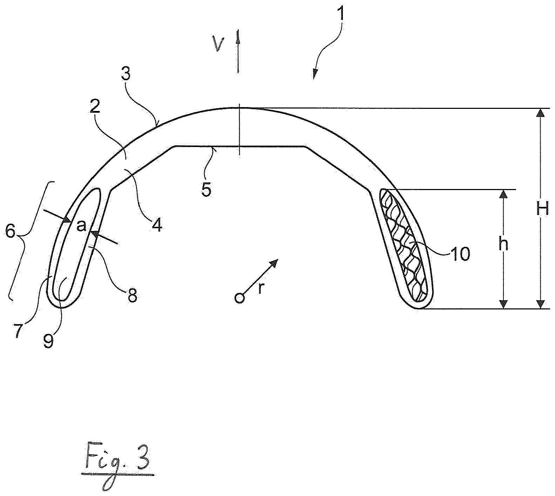

In Figur 1 ist ein Acetabulum 11 schematisch zu erkennen, in welches ein Teil einer Hüftgelenk-Endoprothese, nämlich eine metallische Hüftpfanne 1 implantiert wurde. In der Zusammenschau mit den Figuren 2 und 3 ergibt sich, dass die Hüftpfanne 1 einen äußeren Pfannenabschnitt 2 aufweist, der eine konvexe Oberfläche 3 hat. Diese kontaktiert im implantierten Zustand das Acetabulum 11. Weiterhin hat die Hüftpfanne 1 einen inneren Pfannenabschnitt 4, der im Wesentlichen eine konkave Oberfläche 5 hat und damit einen in etwa halbkugelförmigen Raum umschließt, in dem ein (nicht dargestelltes) Inlay der Hüftgelenk-Endoprothese angeordnet wird (zum Inlay finden sich unten noch nähere Erläuterungen).

In den Figuren 1 bis 3 dargestellt ist ein Radialschnitt durch die Hüftpfanne 1, d. h. ein Schnitt durch die Hüftpfanne 1 entlang eines Großkreises der halbkugelartigen Struktur der Hüftpfanne 1. Demgemäß ergibt sich eine radiale Richtung r von einem gedachten Mittelpunkt der halbkugelartigen Struktur der Hüftpfanne 1. Ferner ist eine vertikale Richtung V eingetragen, die im Wesentlichen die Lage angibt, wenn die Hüftpfanne 1 auf einer ebenen Fläche aufliegt.

In der Zusammenschau der beiden Figuren 1 und 2 lässt sich auch der erzeugte "Pressfit" ersehen, der vorliegt, wenn die Hüftpfanne 1 in das Acetabulum 11 eingeschlagen ist. In Figur 2 ist die Hüftpfanne zunächst noch nicht eingeschlagen und lediglich an das Acetabulum 11 angesetzt, woraus ersichtlich ist, dass hier ein radiales Übermaß der Hüftpfanne 1 relativ zum Aufnahmeraum im Acetabulum 11 vorliegt. In Figur 1 ist der eingeschlagene Zustand zu sehen, gemäß dem nun die Hüftpfanne 1 mit einer gewünschten Vorspannung im Acetabulum 11 angeordnet ist.

Am oberen Pol der Hüftpfanne 1 ist nach dem Einschlagen zumeist noch etwas Spiel vorhanden, da postoperativ mit Aufnahme der vollen Belastung eine gewisse Setzung der Hüftpfanne 1 zu erwarten ist.

Beim Einschlagvorgang ist nun angestrebt, dass sich lediglich die laterale Außenschale (Wandungsabschnitt 7 des äußeren Pfannenabschnitt 2, s. unten) verformt, während die mediale Innenschale (Wandungsabschnitt 8 des inneren Pfannenabschnitt 4, s. unten) sich möglichst wenig verformt.

Um dies zu erreichen, ist vorgesehen, dass über einen Oberflächenabschnitt 6 der Hüftpfanne 1 der äußere Pfannenabschnitt 2 relativ zum inneren Pfannenabschnitt 4 in radiale Richtung r federelastisch gestaltet ist. Hierzu wird zunächst auf Figur 3 Bezug genommen, aus der hervorgeht, dass sich der Oberflächenabschnitt 6 über eine Höhe h erstreckt und in diesem Bereich doppelwandig ausgebildet ist, d. h. er weist einen Wandungsabschnitt 7 des äußeren Pfannenabschnitt 2 und einen Wandungsabschnitt 8 des inneren Pfannenabschnitts 4 auf. Zwischen den beiden Wandungsabschnitten 7 und 8 ist ein Hohlraum 9 ausgebildet, der in Umfangsrichtung über die gesamte Hüftpfanne 1 umläuft. Angegeben ist ein radialer Abstand a über den die beiden Wandungsabschnitte 7 und 8 voneinander beanstandet sind.

Zu sehen ist auch, dass die Höhe h des insoweit federelastisch gestalteten Oberflächenabschnitts 6 nur einen Teil der gesamten Höhe H der Hüftpfanne 1 ausmacht; die diesbezüglich bevorzugte Obergrenze sind 60 % der Höhe H.

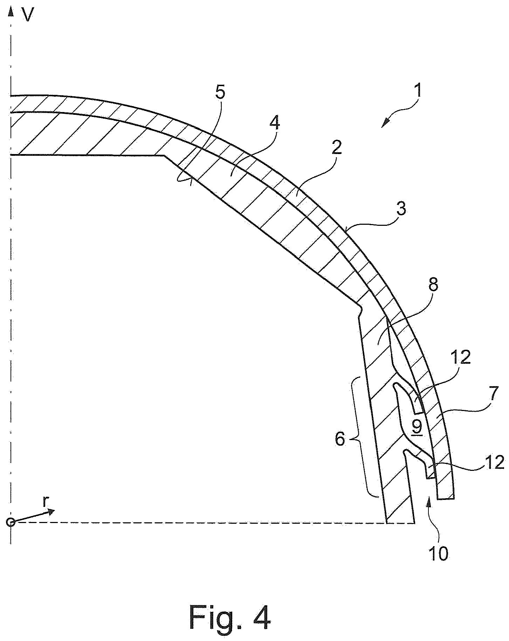

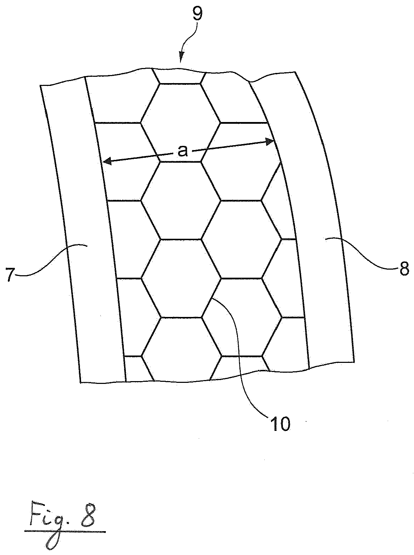

Die Hüftpfanne 1 ist durch ein additives Fertigungsverfahren, insbesondere durch 3-D-Druck, hergestellt, so dass der Hohlraum 9 im Inneren der einstückigen Struktur der Hüftpfanne 1 leicht ausgebildet werden kann (siehe die nachfolgende Figur 8). Der Hohlraum kann sich allerdings auch durch eine zweiteilige Ausbildung der Hüftpfanne 1 ergeben (siehe die nachfolgenden Figuren 4 bis 7).

In den Figuren 1 bis 3 sind dabei verschiedene, teils vorbekannte Möglichkeiten vorgesehen, wie in Bezug auf die Einstellung der Federelastizität zwischen den beiden Wandungsabschnitten 7 und 8 vorgegangen werden kann.

In der linken Bildhälfte von Figur 2 wie auch in der linken Bildhälfte von Figur 3 ist der Hohlraum 9 vollständig frei, d. h. leer. Damit ergibt sich insoweit eine hohe Elastizität zwischen dem äußeren Wandungsabschnitt 7 und dem inneren Wandungsabschnitt 8.

In Figur 1 und auch in der rechten Bildhälfte von Fig. 2 ist indes zu sehen, dass im Hohlraum 9 ein federelastisches Element 10 angeordnet ist, welches erfindungsgemäß eine Wabenstruktur hat, wie sie in Figur 8 nochmals im Detail wiedergegeben ist. Durch das federelastische Element 10 kann die Federelastizität zwischen den beiden Wandungsabschnitten 7, 8 beeinflusst werden, wobei abhängig von der gewählten Struktur des federelastischen Elements 10 die Federrate (Federkonstante) eingestellt werden kann.

Insoweit wird klar, dass es der genannte Hohlraum 9 nicht zwingend macht, dass er gänzlich frei von Material ist; er wird im Rahmen der vorliegenden Beschreibung auch dann als Hohlraum bezeichnet, wenn sich in ihm das federelastische Element 10 befindet. Das federelastische Element 10 ist gemäß der Ausführungsform nach Figur 8 einstückig mit dem beidseitig angeordneten Schalenmaterial der Hüftpfanne 1 ausgebildet, was in einfacher Weise per additiver Fertigung erfolgen kann.

In der rechten Bildhälfte von Figur 3 ist eine alternative, allerdings nicht erfindungsgemäße Ausgestaltung des federelastischen Elements 10 zu sehen, wobei hier eine wellenförmige Struktur vorgesehen ist. Wiederum gilt, dass durch die Gestaltung des federelastischen Elements 10 die Federrate eingestellt werden kann.

Hinsichtlich der Figuren 2 und 3 sei angemerkt, dass hier lediglich zur Illustration eine unterschiedliche Gestaltung des Hohlraums 9 skizziert wurde. Im Regelfall wird der zirkulär umlaufende Hohlraum 9 stets gleichförmig ausgebildet sein, d. h. entweder hohl oder mit einer einheitlichen Struktur des federelastischen Elements 10 versehen sein.

Denkbar ist allerdings grundsätzlich auch, zur Erzeugung eines anisotropen Elastizitätsverhaltens über unterschiedliche Umfangsabschnitte der Hüftpfanne 1 eine verschiedene Gestaltung des Hohlraums 9 vorzusehen.

Ein bevorzugtes Ausführungsbeispiel der Erfindung ist in den Figuren 4 bis 7 dargestellt.

Die Hüftpfanne 1 ist hier zweiteilig ausgebildet, d. h. der äußere Pfannenabschnitt 2 und der innere Pfannenabschnitt 4 bestehen aus zwei zusammengefügten Teilen. Infolge der Möglichkeiten der additiven Fertigung ist es allerdings auch möglich, die Hüftpfanne 1 auch hier einstückig auszubilden.

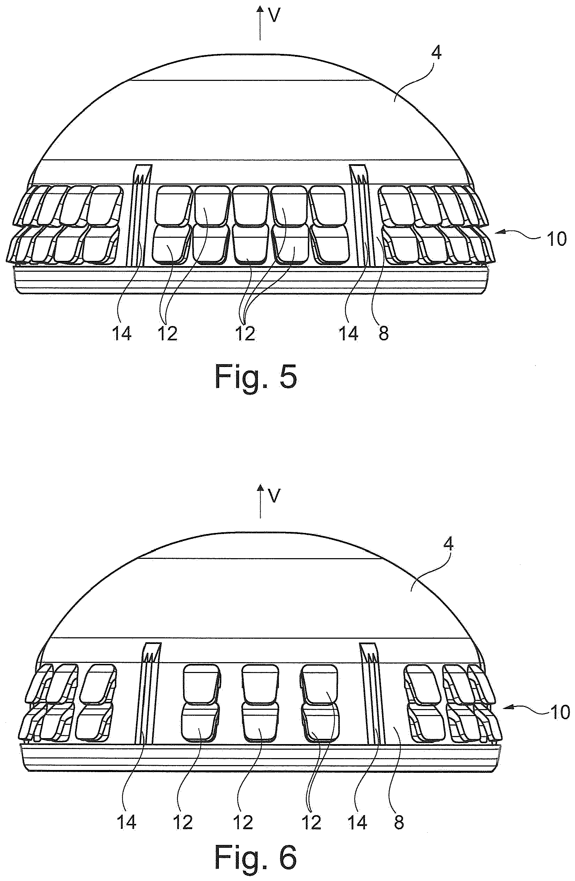

Die Elastizität im Oberflächenabschnitt 6 wird hier dadurch erzeugt, dass in dem Hohlraum 9 zwischen dem Wandungsabschnitt 7 des äußeren Pfannenabschnitt 2 und dem Wandungsabschnitt 8 des inneren Pfannenabschnitts 4 eine Anzahl Federlamellen 12 angeordnet ist. Das federelastische Element 10 wird hier also durch Federlamellen 12 gebildet, die um den Umfang der Hüftpfanne 1 verteilt angeordnet sind; dies geht aus den Figuren 5 und 6 für zwei unterschiedliche Ausgestaltungen hervor.

Dabei sind die Federlamellen 12 aus dem Material des Wandungsabschnitts 8 ausgebildet, was wiederum in vorteilhafter Weise durch additive Fertigung erfolgen kann.

Aus Figur 5 geht hervor, dass hier ca. 30 Federlamellen 12 um den Umfang des inneren Pfannenabschnitts 4 platziert sind, während Figur 6 nur ca. 18 Federlamellen 12 vorsieht. In beiden Fällen gilt, dass zwei Reihen Federlamellen 12 übereinander vorgesehen sind, wie es sich aus den Figuren 4 bis 7 sofort ergibt.

Zu erwähnen sind weiterhin Versteifungselemente 14, wobei über den Umfang des inneren Pfannenabschnitts 4 vier bis sechs solche Versteifungselemente 14 vorgesehen sind. Sie bilden vertikale Verstrebungen zur Versteifung der Inlaykupplung.

Insbesondere aus den Figuren 5 und 6 wird weiter ersichtlich, dass die Form der Federlamellen, betrachtet in radiale Richtung r, im Wesentlichen rechteckig bzw. trapezförmig ist. Die Eckbereiche sind dabei bevorzugt ausgerundet gestaltet (Ausrundungsradius ca. 0,5 bis 1,0 mm).

Der äußere Pfannenabschnitt 2 kann als Halbkugelschale aus homogenem Material gestaltet sein. Möglich ist es aber auch, dass nur der Bereich, der die Federlamellen 12 abdeckt, aus kompaktem Material ausgebildet ist, während der darüber liegende Bereich (siehe Figur 4) aus porösem Material besteht.

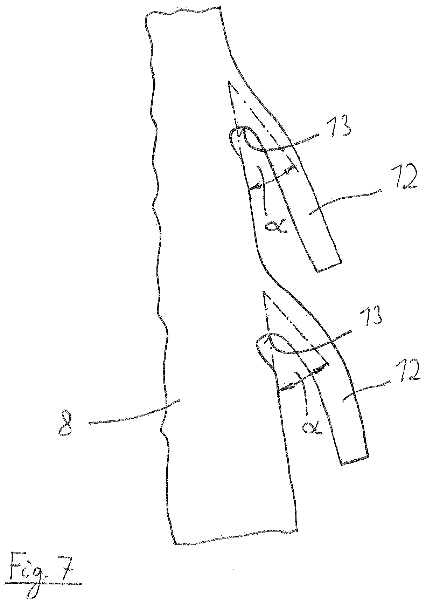

In Figur 7 ist die Gestaltung der Federlamellen 12 noch einmal näher dargestellt. Hieraus ergibt sich zunächst die direkte Ausformung der Federlamellen 12 aus dem Material des Wandungsabschnitts 8. Die Federlamellen 12 treten dabei unter einem Winkel α zur Oberfläche des Wandungsabschnitts 8 aus, wobei dieser Winkel bevorzugt zwischen 20° und 35° liegt. Nach dem Austritt der Federlamelle 12 aus der Oberfläche des Wandungsabschnitts 8 flacht diese ab, d. h. es liegt eine leicht gebogene Struktur vor, wie sie aus Figur 7 ersichtlich ist.

Zur Stabilität und Festigkeit der Federlamelle 12 am Wandungsabschnitt 8 ist im Grund eine Ausrundung 13 vorgesehen. Der Radius dieser Ausrundung 13 liegt zumeist zwischen 0,2 mm und 1,0 mm.

Die Gestaltung des Hohlraums 9 sowie der in der Regel vorgesehenen federelastischen Elemente 10 wird fachmännisch so vorgenommen, dass eine gewünschte Federrate zwischen den Wandungsabschnitt 7 und 8 vorliegt. Generell ist dabei angestrebt, die Außenschale (d. h. den Wandungsabschnitt 7 des äußeren Pfannenabschnitts 2) mit einer gewünschten Nachgiebigkeit zu versehen, während die Innenschale (d. h. den Wandungsabschnitt 8 des inneren Pfannenabschnitts 4) möglichst formstabil zu halten, womit die oben erläuterten Vorteile erreicht werden können.

Die zum Einsatz kommenden Inlays sind zumeist modular gestaffelt und werden in verschiedenen Größenabstufungen vorgehalten. Hierbei sind unterschiedliche Hüftpfannen-Größen relevant, die das Inlay aufnehmen. Weiterhin können verschiedene Außendurchmesser bzw. Außenkonturen vorgesehen sein, die exakt die Innendurchmesser bzw. Innenkontur der aufnehmenden metallischen Hüftpfanne passen müssen. Bei den Inlays werden üblicherweise mehrere Hüftkopf-Aufnahmedurchmesser mit einer identischen Außenkontur ausgestattet, so dass sie in ein und dieselbe metallische Hüftpfanne passen. Daraus ergeben sich dann natürlich entsprechende unterschiedliche Wandstärken des Inlays.

Betreffend die Hüftpfanne und das Inlay sind verschiedene Werkstoffkombinationen möglich. Das Inlay kann aus PE-Kunststoff oder Keramik bestehen. Folgende Materialpaarungen sind möglich bzw. gebräuchlich: Metall auf Polyäthylen (englisch abgekürzt "MoP"), Metall auf Keramik "MoC"), Keramik auf Polyäthylen ("CoP") und Keramik auf Keramik ("CoC").

### Bezugszeichenliste:

- 1: Hüftpfanne
- 2: äußerer Pfannenabschnitt
- 3: konvexe Oberfläche
- 4: innerer Pfannenabschnitt
- 5: konkave Oberfläche
- 6: Oberflächenabschnitt
- 7: Wandungsabschnitt des äußeren Pfannenabschnitts
- 8: Wandungsabschnitt des inneren Pfannenabschnitts
- 9: Hohlraum
- 10: federelastisches Element
- 11: Acetabulum
- 12: Federlamelle
- 13: Ausrundung
- 14: Versteifungselement

- r: radiale Richtung
- a: radialer Abstand
- h: Höhe des federelastisch gestalteten Oberflächenabschnitts
- H: gesamte Höhe der Hüftpfanne
- V: vertikale Richtung
- α: Winkel

## Patentansprüche

1. Hüftpfanne (1) einer Hüftgelenk-Endoprothese, umfassend einen äußeren Pfannenabschnitt (2) mit einer konvexen Oberfläche (3) zur Kontaktnahme mit dem Acetabulum (11) und mit einem inneren Pfannenabschnitt (4) mit einer konkaven Oberfläche (5) zur Kontaktnahme mit einem Inlay der Hüftgelenk-Endoprothese, wobei zumindest über einen Oberflächenabschnitt (6) der Hüftpfanne (1) der äußere Pfannenabschnitt (2) relativ zum inneren Pfannenabschnitt (4) in eine radiale Richtung (r) federelastisch gestaltet ist,
wobei die Federelastizität durch einen Wandungsabschnitt (7) des äußeren Pfannenabschnitts (2) gebildet wird, der zu einem Wandungsabschnitt (8) des inneren Pfannenabschnitts (4) unter Bildung eines Hohlraums (9) mit radialem Abstand (a) verläuft,
wobei im Hohlraum (9) mindestens ein federelastisches Element (10) angeordnet ist, welches eine Federwirkung in radiale Richtung (r) zwischen dem Wandungsabschnitt (7) des äußeren Pfannenabschnitts (2) und dem Wandungsabschnitt (8) des inneren Pfannenabschnitts (4) erzeugt,
**dadurch gekennzeichnet,**
**dass** das federelastische Element (10) durch eine Anzahl Federlamellen (12) gebildet wird, die am einen Wandungsabschnitt (8) angeformt sind und die auf der Oberfläche des anderen Wandungsabschnitts (7) anliegen, wobei die Federlamellen (12) um den Umfang des Wandungsabschnitts (8) umlaufend angeordnet sind, wobei um den Umfang des Wandungsabschnitts (8) zwischen 14 und 40 Federlamellen (12) angeordnet sind, wobei die Federlamellen (12), aus radialer Richtung (r) betrachtet, eine rechteckige oder trapezförmige Grundkontur aufweisen und wobei die Federlamellen (12) aus dem Material eines Wandungsabschnitts direkt ausgeformt sind,
oder
**dass** das federelastische Element (10) in einem Radialschnitt eine waben- oder fachwerkartige Struktur aufweist.

2. Hüftpfanne nach Anspruch 1, **dadurch gekennzeichnet, dass** die Federlamellen (12) am Wandungsabschnitt (8) des inneren Pfannenabschnitts (4) angeformt sind und auf der Oberfläche des Wandungsabschnitts (7) des äußeren Pfannenabschnitts (2) anliegen.

3. Hüftpfanne nach Anspruch 1, **dadurch gekennzeichnet, dass** um den Umfang des Wandungsabschnitts (8) zwischen 16 und 35 Federlamellen (12) angeordnet sind.

4. Hüftpfanne nach einem der Ansprüche 1 bis 3, **dadurch gekennzeichnet, dass** in vertikale Richtung (V) der Hüftpfanne (1) versetzt mindestens zwei Reihen Federlamellen (12) angeordnet sind.

5. Hüftpfanne nach einem der Ansprüche 1 bis 4, **dadurch gekennzeichnet, dass** die Federlamellen (12) mit ihrem in vertikale Richtung (V) der Hüftpfanne (1) oberen oder unteren Endbereich mit dem Wandungsabschnitt (8) verbunden sind.

6. Hüftpfanne nach einem der Ansprüche 1 bis 5, **dadurch gekennzeichnet, dass** die Federlamellen (12) unter einem Winkel (α) zwischen 15° und 45°, vorzugsweise zwischen 20° und 35°, zur Oberfläche des Wandungsabschnitts (8) austreten.

7. Hüftpfanne nach Anspruch 6, **dadurch gekennzeichnet, dass** im Übergangsbereich zwischen Wandungsabschnitt (8) und Federlamelle (12) eine Ausrundung (13) ausgebildet ist, vorzugsweise mit einem Radius zwischen 0,1 mm und 1,5 mm, besonders bevorzugt zwischen 0,2 mm und 1,0 mm.

8. Hüftpfanne nach einem der Ansprüche 1 bis 7, **dadurch gekennzeichnet, dass** in Umfangsrichtung des Wandungsabschnitts (8) zwischen mindestens zwei Federlamellen (12) ein sich in vertikale Richtung (V) erstreckendes Versteifungselement (14) am Wandungsabschnitt (8) angeordnet ist.

9. Hüftpfanne nach einem der Ansprüche 1 bis 8, **dadurch gekennzeichnet, dass** sich der federelastisch gestaltete Oberflächenabschnitt (6) auf eine Höhe (h) beschränkt, die maximal 60 %, vorzugsweise maximal 50 %, der gesamten Höhe (H) der Hüftpfanne (1) beträgt.

10. Hüftpfanne nach einem der Ansprüche 1 bis 9, **dadurch gekennzeichnet, dass** der federelastisch gestaltete Oberflächenabschnitt (6) um den gesamten Umfang der Hüftpfanne verläuft.

11. Hüftpfanne nach einem der Ansprüche 1 bis 10, **dadurch gekennzeichnet, dass** sie aus Metall besteht.

12. Hüftpfanne nach einem der Ansprüche 1 bis 11, **dadurch gekennzeichnet, dass** sie durch einen additiven Fertigungsprozess hergestellt ist.

## Claims

1. Acetabular cup (1) of a hip joint endoprosthesis, comprising an outer cup section (2) with a convex surface (3) for contacting the acetabulum (11) and with an inner cup section (4) with a concave surface (5) for contacting an inlay of the hip joint endoprosthesis, wherein at least along a surface portion (6) of the acetabular cup (1) the outer cup section (2) is made resilient relative to the inner cup section (4) in a radial direction (r),
wherein the spring elasticity is formed by a wall portion (7) of the outer cup section (2) extending to a wall portion (8) of the inner cup section (4) forming a cavity (9) with radial clearance (a),
wherein at least one spring-elastic element (10) is arranged in the cavity (9), which element produces a spring effect in the radial direction (r) between the wall portion (7) of the outer cup section (2) and the wall portion (8) of the inner cup section (4),
**characterized in**
**that** the spring-elastic element (10) is formed by a number of spring lamellae (12) which are formed on one wall portion (8) and which bear on the surface of the other wall portion (7), wherein the spring lamellae (12) being arranged circumferentially around the circumference of the wall portion (8), wherein between 14 and 40 spring lamellae (12) are arranged around the circumference of the wall portion (8), wherein the spring lamellae (12), viewed from the radial direction (r), have a rectangular or trapezoidal basic contour and wherein the spring lamellae (12) are formed directly from the material of a wall portion,
or
**that** the spring-elastic element (10) has a honeycomb-like or lattice-like structure in a radial section.

2. Acetabular cup according to claim 1, **characterized in that** the spring lamellae (12) are formed on the wall portion (8) of the inner cup section (4) and rest on the surface of the wall portion (7) of the outer cup section (2).

3. Acetabular cup according to claim 1, **characterized in that** between 16 and 35 spring lamellae (12) are arranged around the circumference of the wall portion (8).

4. Acetabular cup according to one of claims 1 to 3, **characterized in that** at least two rows of spring lamellae (12) are arranged offset in the vertical direction (V) of the acetabular cup (1).

5. Acetabular cup according to one of claims 1 to 4, **characterized in that** the spring lamellae (12) are connected to the wall portion (8) with their upper or lower end region in the vertical direction (V) of the acetabular cup (1).

6. Acetabular cup according to one of claims 1 to 5, **characterized in that** the spring lamellae (12) emerge at an angle (α) of between 15° and 45°, preferably between 20° and 35°, to the surface of the wall portion (8).

7. Acetabular cup according to claim 6, **characterized in that** a fillet (13) is formed in the transition region between the wall portion (8) and the spring lamella (12), preferably with a radius between 0.1 mm and 1.5 mm, particularly preferably between 0.2 mm and 1.0 mm.

8. Acetabular cup according to one of claims 1 to 7, **characterized in that** a stiffening element (14) extending in the vertical direction (V) is arranged on the wall portion (8) in circumferential direction of the wall portion (8) between at least two spring lamellae (12).

9. Acetabulum according to one of claims 1 to 8, **characterized in that** the resiliently designed surface portion (6) is limited to a height (h) which is at most 60 %, preferably at most 50 %, of the total height (H) of the acetabulum (1).

10. Acetabular cup according to one of claims 1 to 9, **characterized in that** the resiliently designed surface portion (6) extends around the entire circumference of the acetabular cup.

11. Acetabular cup according to one of claims 1 to 10, **characterized in that** it is made of metal.

12. Acetabular cup according to one of claims 1 to 11, **characterized in that** it is produced by an additive manufacturing process.

## Revendications

1. Cavité cotyloïde (1) d'une endoprothèse de l'articulation de la hanche, ladite cavité cotyloïde comprenant une portion de cavité externe (2) pourvue d'une surface convexe (3) destinée à venir en contact avec l'acétabulum et une portion de cavité interne (4) pourvue d'une surface concave (5) destinée à venir en contact avec un insert de l'endoprothèse de l'articulation de la hanche, la portion de cavité externe (2) étant conçue pour être élastique dans une direction radiale (r) par rapport à la portion de cavité interne (4) au moins sur une portion de surface (6) de la cavité cotyloïde (1),
l'élasticité étant formée par une portion de paroi (7) de la portion de cavité externe (2) qui s'étend à une distance radiale (a) d'une portion de paroi (8) de la portion de cavité interne (4) pour former une cavité (9), au moins un élément élastique (10) étant agencé dans la cavité (9), lequel produit un effet de ressort dans la direction radiale (r) entre la portion de paroi (7) de la portion de cavité externe (2) et la portion de paroi (8) de la portion de cavité interne (4),
**caractérisée en ce que**
l'élément élastique (10) est formé par un nombre de lamelles élastiques (12) qui sont formées sur une portion de paroi (8) et qui reposent sur la surface de l'autre portion de paroi (7), les lamelles élastiques (12) étant agencées de manière périphérique autour de la circonférence de la portion de paroi (8), entre 14 et 40 lamelles élastiques (12) étant agencées autour de la circonférence de la section de paroi (8), les lamelles élastiques (12), telles que vues dans la direction radiale (r), présentant un contour de base rectangulaire ou trapézoïdal, et les lamelles élastiques (12) étant formées directement à partir du matériau d'une portion de paroi,
ou
**en ce que** l'élément élastique (10) présente une structure en nid d'abeille ou en treillis dans une coupe radiale.

2. Cavité cotyloïde selon la revendication 1, **caractérisée en ce que** les lamelles élastiques (12) sont formées sur la portion de paroi (8) de la portion de cavité interne (4) et reposent sur la surface de la portion de paroi (7) de la portion de cavité externe (2).

3. Cavité cotyloïde selon la revendication 1, **caractérisée en ce qu'**entre 16 et 35 lamelles élastiques (12) sont agencées autour de la circonférence de la portion de paroi (8).

4. Cavité cotyloïde selon l'une quelconque des revendications 1 à 3, **caractérisé en ce qu'**au moins deux rangées de lamelles élastiques (12) sont agencées de manière décalée dans la direction verticale (V) de la cavité cotyloïde (1).

5. Cavité cotyloïde selon l'une quelconque des revendications 1 à 4, **caractérisée en ce que** les lamelles élastiques (12) sont reliées à la portion de paroi (8) par leur zone d'extrémité supérieure ou inférieure dans la direction verticale (V) de la cavité cotyloïde (1).

6. Cavité cotyloïde selon l'une quelconque des revendications 1 à 5, **caractérisée en ce que** les lamelles élastiques (12) sortent à un angle (a) compris entre 15° et 45°, de préférence entre 20° et 35°, par rapport à la surface de la portion de paroi (8).

7. Cavité cotyloïde selon la revendication 6, **caractérisé en ce que** dans la zone de transition entre la portion de paroi (8) et la lamelle élastique (12), un arrondi (13) est formé, de préférence avec un rayon compris entre 0,1 mm et 1,5 mm, de manière particulièrement préférée entre 0,2 mm et 1,0 mm.

8. Cavité cotyloïde selon l'une quelconque des revendications 1 à 7, **caractérisée en ce qu'**un élément de renforcement (14) s'étendant dans la direction verticale (V) est agencé sur la portion de paroi (8) dans la direction circonférentielle de la portion de paroi (8) entre au moins deux lamelles élastiques (12).

9. Cavité cotyloïde selon l'une quelconque des revendications 1 à 8, **caractérisée en ce que** la portion de surface élastique (6) est limitée à une hauteur (h) qui représente 60 % maximum, de préférence 50 % maximum, de la hauteur totale (H) de la cavité cotyloïde (1).

10. Cavité cotyloïde selon l'une quelconque des revendications 1 à 9, **caractérisée en ce que** la portion de surface élastique (6) s'étend sur toute la circonférence de la cavité cotyloïde.

11. Cavité cotyloïde selon l'une quelconque des revendications 1 à 10, **caractérisée en ce qu'**elle est en métal.

12. Cavité cotyloïde selon l'une quelconque des revendications 1 à 11, **caractérisée en ce qu'**elle est réalisée par un processus de fabrication additive.
